# EUROPEAN PATENT APPLICATION

(11) **EP 4 810 090 A1**
(43) Date of publication of application: **23.09.2026**
(21) Application number: 25823729.6
(22) Date of filing: 21.11.2025
(51) Int. Cl.: A61M 16/06, A62B 18/08

(54) **HEADGEAR ASSEMBLY, MANUFACTURING METHOD FOR HEADGEAR ASSEMBLY, AND VENTILATION MASK SYSTEM**

(30) Priority: 31.12.2024 CN 202411998195; 31.12.2024 CN 202411999561
(71) Applicant: BMC Medical Co., Ltd., Beijing 100142 (CN)
(72) Inventor: LIU, Lina, Beijing 100142 (CN); WANG, Yajie, Beijing 100142 (CN); LI, Zhi, Beijing 100142 (CN); ZHOU, Mingzhao, Beijing 100142 (CN); ZHUANG, Zhi, Beijing 100142 (CN)
(74) Representative: Grünecker Patent- und Rechtsanwälte PartG mbB
(86) International application number: PCT/CN2025/136779
(87) International publication number: WO 2026/144638

(57) **Abstract**

The present disclosure relates to the technical field of ventilation mask systems, and relates to a headgear assembly, a manufacturing method for a headgear assembly, and a ventilation mask system. The headgear assembly provided in the present disclosure is configured to secure a mask body of a ventilation mask system to the user's head. The headgear assembly comprises a strap body, and the strap body comprises a main headstrap and connecting straps connected to the main headstrap. The main headstrap is located at an occipital region of the user when worn, and the connecting straps are configured to connect to the mask body of the ventilation mask system; and the main headstrap has a lowest point and is recessed upward and toward the connecting straps from the lowest point, where the main headstrap is integrally of a symmetrical structure, or at least part of local straps on the main headstrap are each of a symmetrical structure, and the symmetrical structure has a boundary line or a local boundary line configured as one of a parabola, a hyperbola, a circle, and an ellipse.

## Description

### Cross-Reference to Related Applications

The present disclosure claims priorities of Chinese patent application CN202411998195.3, filed on December 31, 2024, entitled "HEADGEAR ASSEMBLY, MANUFACTURING METHOD FOR HEADGEAR ASSEMBLY, AND VENTILATION MASK SYSTEM" and Chinese patent application CN202411999561.7, filed on December 31, 2024, entitled "HEADGEAR ASSEMBLY, MANUFACTURING METHOD, AND MASK SYSTEM", the entire contents of which are incorporated herein by reference.

### Technical Field

The present disclosure relates to the technical field of ventilation mask systems, and in particular, to a headgear assembly, a manufacturing method for the headgear assembly, and a ventilation mask system.

### Background

Respiratory diseases include obstructive sleep apnea (OSA), Cheyne-Stokes respiration (CSR), respiratory insufficiency, obesity hypoventilation syndrome (OHS), chronic obstructive pulmonary disease (COPD), neuromuscular disease (NMD), and chest wall disease, etc. Mechanical ventilation can be effectively used for treating the aforementioned respiratory diseases. Mechanical ventilation includes invasive ventilation, in which intubation therapy is performed using an invasive ventilation user interface, and non-invasive ventilation, in which mask-based therapy is performed using a non-invasive ventilation user interface. A mask is a type of user interface for a non-invasive ventilation treatment system. By wearing the mask, gas delivered by a breathing device is conveyed into the user's respiratory tract through the user's nose and/or mouth. The mask forms a seal against a region of the user's face to facilitate treatment under an appropriate positive pressure.

A headgear assembly is worn on the user's head for securing a main body of the mask to the user's face. On the one hand, a headgear ensures sealing between the main body of the mask and the user's face, and therefore the headgear needs to have a certain strength; and on the other hand, the headgear contacts the user's skin, and therefore the headgear needs to have a certain softness and breathability so as to improve wearing comfort.

WO 2013050920 A1 discloses a method of geometrically forming a portion of a headgear to conform to a selected portion of the head of an intended user, the method including determining a triangular region on a helmet portion that generally coincides with the selected portion of the head of the intended user. The triangular region is defined by a first side, a second side, and a third side; and the method also includes forming a curved cup-shaped region in a portion of the headgear by putting the second and third sides of the triangular region together and joining them together.

JP 7210289 B2 discloses a method for creating a customized headgear including a plurality of physical features and usable with a mask assembly that delivers a flow of breathing gas to the user's airway. The method includes the following steps: receiving one or more parameters relating to the user's head, a length of one of the plurality of physical features, and an angle between a pair of physical features among the plurality of physical features; transmitting some of the parameters to one or more algorithms to determine at least one of at least one main body, where the body, when formed, may be configured to assemble at least a portion of the headgear; and outputting a pattern usable for implementing the formation of at least a portion of the main body from at least a first sheet of at least a first material.

US 6776161 B2 discloses a headgear for a respirator, which includes a head plate (1) that is elastic in an X direction and that is substantially inelastic in a Y direction. Front straps (2), temple straps (3) and neck straps (4) are fastened to the head plate, and only the temple straps and the neck straps are extensible. Strap buckles (5) provided for fastening the front straps and the temple straps to a mask base are rigidly adjusted according to head size so that a mask is already correctly positioned and preliminarily fixed when put on and only the neck straps need to be readjusted. The headgear can be adapted to different head shapes and sizes and allows for high wearing comfort. The respirator can be quickly and easily put on.

CN 104524679 A discloses a headgear for a mask, which comprises a first strap (660) configured to engage the back of the user's head and extend along either side of the user's parietal bone to positions behind the user's ears, and is substantially circular or oval during use. At least a portion of the first strap is substantially inextensible. The headgear also includes at least one second strap (620, 630) for detachably connecting the first strap to the mask. The second strap may be more extensible than the first strap. At least a portion of the first strap is self-supporting such that the headgear maintains a three-dimensional shape when not in use. The substantially inextensible portion of the first strap is configured to elastically return to a predetermined shape when not in use. An arcuate region comprises a first portion configured to be substantially aligned parallel to the top of the user's head, and a second portion configured to be substantially aligned along the posterior surface of the user's head.

It is difficult to quantify and visualize dimensional data of existing headgear assemblies, resulting in large dimensional differences among the headgear assemblies and making it difficult to realize fully automated production of the headgear assemblies compatible with strength, softness and breathability.

### SUMMARY

The present disclosure provides a headgear assembly, a manufacturing method for the headgear assembly, and a ventilation mask system to solve at least one of the above technical problems.

The present disclosure provides a headgear assembly for securing a mask body of a ventilation mask system to the user's head. The headgear assembly includes a strap body, which includes a main headstrap and connecting straps connected to the main headstrap. The main headstrap is located at an occipital region of the user when worn, and the connecting straps are configured to connect to the mask body of the ventilation mask system.

In an implementation, the main headstrap has a lowest point and is recessed upward and toward the connecting straps from the lowest point, where the main headstrap is integrally of a symmetrical structure, or at least part of local straps on the main headstrap are each of a symmetrical structure, and the symmetrical structure has a boundary line or a local boundary line configured as one of a parabola, a hyperbola, a circle, and an ellipse.

According to the headgear assembly of the present disclosure, the main headstrap can form a band-shaped structure covering the user's head. Due to its recessed structure, the contact area between the main headstrap and the user's head can be reduced, and a ventilation gap can be formed between the main headstrap and the user's head, thereby improving the wearing comfort of the user; in addition, by forming the boundary line or the local boundary line of the headgear assembly with reference to the structure of the user's head, quantifiable and visualized reliable data is provided for the production of the headgear assembly, which can reduce dimensional differences of the headgear assembly and is conducive to realizing fully automated production of the headgear assembly.

In an implementation, the boundary line is a symmetrical center line or an outer contour line of the entire main headstrap, or the local boundary line is a symmetrical center line or an outer contour line of at least part of the local straps on the main headstrap.

According to the headgear assembly of the present disclosure, by configuring the boundary line that is integrally symmetrical or partially symmetrical, the headgear assembly can be obtained by extending outward from one side of the boundary line or by extending outward from both sides of the boundary line.

In an implementation, the strap body is formed of a fabric material, a flexible material, or a foam member that includes a sponge material, where the foam member has a reinforcing portion formed by hot pressing.

According to the headgear assembly of the present disclosure, the strap body formed of the above materials can more easily improve the wearing comfort of the user while meeting the strength requirement to hermetically support the mask body on the user's face.

In an implementation, the reinforcing portion is disposed at any one of the following positions:
at least one end of the strap body in a width direction, the reinforcing portion extending in a length direction of the strap body;
a middle portion of the strap body in the width direction, the reinforcing portion extending in the length direction of the strap body; and
a front surface of the strap body.

According to the headgear assembly of the present disclosure, the reinforcing portion can increase the strength of the strap body, such that the headgear assembly has sufficient strength while maintaining good softness and breathability.

In an implementation, in a thickness direction of the strap body, the strap body includes at least one layer of foam member, and a density of the at least one layer of foam member is greater than or equal to 45 Kg/m³; or in the thickness direction of the strap body, the strap body includes at least two layers of foam members, the at least two layers of foam members are arranged in a stacked manner in the thickness direction, and the density of the foam member located on the front surface of the strap body among the at least two layers of foam members is larger than the density of the foam member located on a back surface of the strap body.

According to the headgear assembly of the present disclosure, when the strap body includes at least one layer of foam member and the density of the at least one layer of foam member is greater than or equal to 45 Kg/m³, the reinforcing portion formed by hot pressing can meet the strength requirement of the headgear assembly; and when the strap body includes at least two layers of foam members, the foam member located on the front surface of the strap body has better strength, which can increase the strength of the strap body, and the foam member located on the back surface of the strap body has better breathability and softness, which can effectively improve the wearing comfort of the user.

In an implementation, the strap body includes a first foam member located on a front surface of the strap body, a second foam member located on a back surface of the strap body, and a third foam member located between the first foam member and the second foam member, where the density of the third foam member is larger than that of the second foam member, and the density of the first foam member is larger than that of the second foam member.

According to the headgear assembly of the present disclosure, the provision of an intermediate foam member enables a more flexible design of the foam members to meet different needs for breathability, softness and strength.

In an implementation, a thickness of the strap body ranges from 3 mm to 6 mm.

According to the headgear assembly of the present disclosure, when the thickness falls within the above range, it can not only meet the requirements of the headgear assembly for breathability, softness and tensile strength, but also avoid the bulkiness caused by an excessive thickness.

In an implementation, the strap body has a tensile strength in the range of 450 Kpa to 900 Kpa.

According to the headgear assembly of the present disclosure, when the tensile strength falls within the above range, the tensile strength can meet the use requirements after the headgear assembly is connected to a mask.

In an implementation, the local straps on the main headstrap are connected to each other by seams or integral molding, and the hardness of connection positions between the local straps is larger than the hardness of non-connection positions.

According to the headgear assembly of the present disclosure, setting the hardness of the connection positions to be higher can avoid winding and bending at seam positions, thereby preventing the service life thereof from being affected.

In an implementation, two or four connecting straps are provided, and the main headstrap and the respective connecting straps are connected to each other by seams or integral molding.

According to the headgear assembly of the present disclosure, the connection by seams, splicing, or integral molding makes the molding methods of the headgear assembly more diversified.

In an implementation, the strap body is configured to be wrapped around a ventilation headstrap tube of the mask body.

According to the headgear assembly of the present disclosure, the headgear assembly is applicable to a mask body configured as a ventilation headstrap tube.

In an implementation, the boundary line or the local boundary line satisfies the following relational expression:
$y = {ax}^{2} + bx + c ,$
where *y* is a value on a *y*-axis in a Cartesian coordinate system *xOy* for the boundary line, and *x* is a value on an x-axis in the Cartesian coordinate system *xOy* for the boundary line; and
*a*, *b* and *c* are all constants, 0 *< a* < 1.2 mm, *b =* 0, and 0 ≤ *c* ≤ 1.2 mm.

In an implementation, a rotation angle *α* between a projection of the boundary line or the local boundary line in a *-yOz* plane of a three-dimensional Cartesian coordinate system *O-xyz* and a -*z* axis is 0°-90°, where a *z*-axis of the three-dimensional Cartesian coordinate system *O-xyz* is a straight line perpendicular to the Cartesian coordinate system *xoy* and passing through an origin O of the Cartesian coordinate system *xoy,* and the three-dimensional Cartesian coordinate system is a right-hand coordinate system.

According to the headgear assembly of the present disclosure, by establishing the Cartesian coordinate system *xOy* and the three-dimensional Cartesian coordinate system *O-xyz* with reference to the structure of the user's head, a mathematical model of the headgear assembly can be constructed. This provides quantifiable and visualized reliable data for the production of the headgear assembly, reduces dimensional differences of the headgear assembly, and enables unification of main dimensions of the headgear assembly.

The present disclosure also provides a ventilation mask system, including a headgear assembly described above and further including a mask body, where the strap body is connected to the mask body.

The present disclosure also provides a manufacturing method for a headgear assembly, including a step of constructing a mathematical model of a main headstrap of a headgear assembly, the step of constructing the mathematical model of the main headstrap of the headgear assembly including the following steps:
establishing a Cartesian coordinate system *xOy,* where an origin *O* of the Cartesian coordinate system *xOy* is a midpoint of a symmetrical part of the user's neck, a *y*-axis of the Cartesian coordinate system *xOy* is a connecting line between a highest point on an occipital region of the user and the midpoint, and an *x*-axis of the Cartesian coordinate system *xOy* is a straight line passing through the origin *O* and perpendicular to the *y*-axis; and
constructing a boundary line of the entire main headstrap or a local boundary line of at least one local strap among a plurality of unitized local straps in the Cartesian coordinate system *xOy;*
where the boundary line or the local boundary line is one of a parabola, a hyperbola, a circle, and an ellipse in the Cartesian coordinate system *xOy.*

In an implementation, the method further includes a step of manufacturing the headgear assembly according to the mathematical model of the headstrap, the step including the following operation steps:
obtaining a foam member by foam molding;
performing hot pressing on at least one end of the foam member in a width direction to obtain a strap body with a reinforcing portion, or performing hot pressing on a front surface of the foam member to obtain a strap body with a reinforcing portion; and
connecting at least one strap body to form the headgear assembly.

Compared with the prior art, the advantages of the present disclosure are as follows: by forming the boundary line or the local boundary line of the headgear assembly with reference to the structure of the user's head, quantifiable and visualized reliable data is provided for the production of the headgear assembly, which can reduce dimensional differences of the headgear assembly and is conducive to realizing fully automated production of the headgear assembly.

### Brief Description of the Drawings

The present disclosure will be described in more detail below, based on embodiments and with reference to the drawings.
FIG. 1 is a schematic structural perspective view of a headgear assembly according to an embodiment of the present disclosure;
FIG. 2 is a schematic structural diagram of a headgear assembly according to an embodiment of the present disclosure;
FIG. 3 is a schematic structural diagram of a headgear assembly as a ventilation headstrap tube according to an embodiment of the present disclosure;
FIG. 4 is a first schematic diagram of a partial structure of the headgear assembly according to an embodiment of the present disclosure;
FIG. 5 is a second schematic diagram of a partial structure of the headgear assembly according to an embodiment of the present disclosure;
FIG. 6 is a third schematic diagram of a partial structure of the headgear assembly according to an embodiment of the present disclosure;
FIG. 7 is a schematic structural diagram of a three-dimensional Cartesian coordinate system according to an embodiment of the present disclosure;
FIG. 8 is a schematic structural diagram of the rear side of the user's head, which shows a highest point M1 on an occipital region of the user;
FIG. 9 is a schematic structural diagram of the rear side of the user's head, which shows a midpoint M2 of a symmetrical part of the user's neck;
FIG. 10 is a curve graph of a boundary line according to an embodiment of the present disclosure;
FIG. 11 is a projection diagram of the boundary line in a plane *-zoy* according to an embodiment of the present disclosure; and
FIG. 12 is a flow diagram of a manufacturing method for the headgear assembly according to an embodiment of the present disclosure.

### List of reference signs:

1. headgear assembly;
11. strap body; 12. reinforcing portion; 13. boundary line;
101. main headstrap; 102. connecting strap; 103. lowest point; 104. top headstrap;
20. ventilation headstrap tube;
2. mask body; 3. ventilation gas pipeline.

### Detailed Description of Embodiments

The present disclosure will be further described below in conjunction with the accompanying drawings.

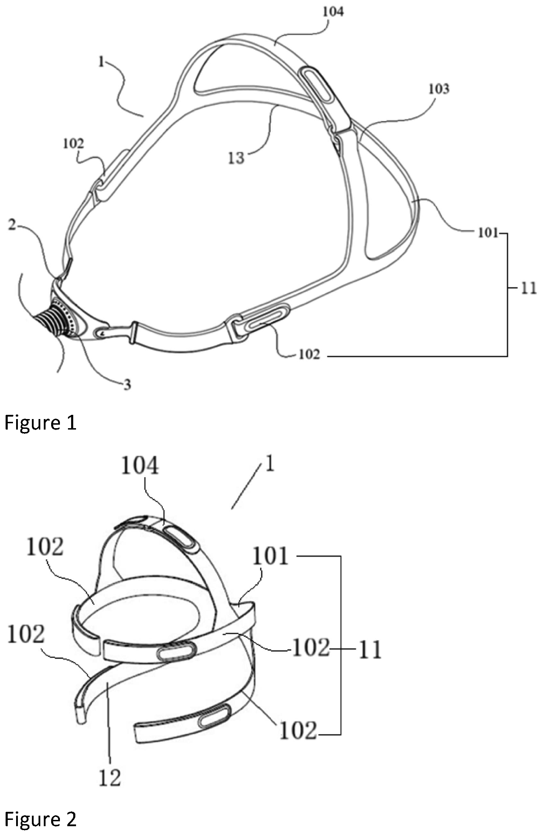

As shown in FIGS. 1-6, a headgear assembly 1 of the present disclosure is configured to secure a mask body 2 of a ventilation mask system to the user's head. The headgear assembly 1 is adapted to be connected to the mask body 2, enabling the user to wear the ventilation mask system on the face via the headgear assembly 1 and providing a tight and stable fixation.

As shown in FIGS. 1 and 2, the headgear assembly 1 includes a strap body 11, and the strap body 11 includes a main headstrap 101 and connecting straps 102 connected to the main headstrap 101. The main headstrap 101 is located at an occipital region of the user when worn, and the connecting straps 102 are configured to connect to the mask body 2 of the ventilation mask system. The main headstrap 101 has a lowest point 103, and the main headstrap 101 is recessed upward and toward the connecting straps 102 from the lowest point 103. Therefore, the main headstrap 101 can form a band-shaped structure covering the user's head, and due to the recessed structure, the contact area between the main headstrap and the user's head can be reduced, and a ventilation gap can be formed between the main headstrap and the user's head, thereby improving the wearing comfort of the user.

As shown in FIGS. 1 and 2, the main headstrap 101 is integrally of a symmetrical structure, and the symmetrical structure has a boundary line configured as one of a parabola, a hyperbola, a circle, and an ellipse. Alternatively, at least part of local straps on the main headstrap 101 are each of a symmetrical structure, and the symmetrical structure has a local boundary line configured as one of a parabola, a hyperbola, a circle, and an ellipse.

The boundary line or the local boundary line is one of a parabola, a hyperbola, a circle, and an ellipse in the Cartesian coordinate system *xOy.*

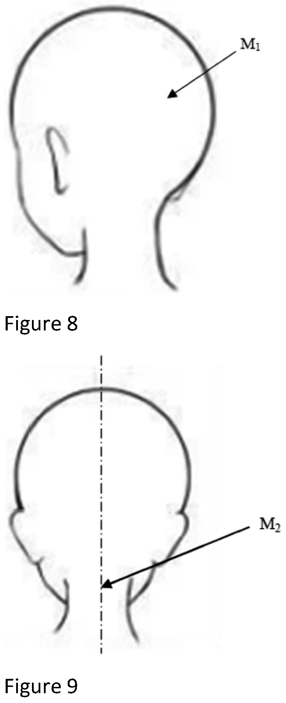

As shown in FIG. 9, when an origin *O* of a Cartesian coordinate system *xOy* is a midpoint M₂ of a symmetrical part of the user's neck when the headgear assembly is worn on the user's head. A *y*-axis of the Cartesian coordinate system *xOy* is a connecting line M₂ between the highest point M₁ on the occipital region of the user and the midpoint. An x-axis of the Cartesian coordinate system *xOy* is a straight line passing through the origin *O* and perpendicular to the *y*-axis.

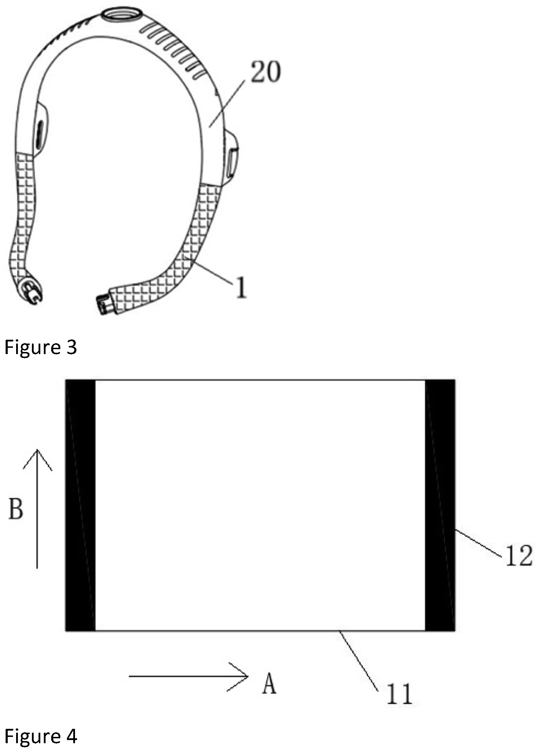

Specifically, the boundary line or the local boundary line may be a parabola, for example, which satisfies the following relational expression:
$y = {ax}^{2} + bx + c ,$
where *y* is a value on the *y*-axis in the Cartesian coordinate system *xOy* for the boundary line, and *x* is a value on the *x*-axis in the Cartesian coordinate system *xOy* for the boundary line. *a*, *b* and *c* are all constants, 0 *< a* < 1.2 mm, *b =* 0, and 0 ≤ *c* ≤ 1.2 mm. As shown in FIG. 4, the functional relational expression of the boundary line or the local boundary line is shown when *a* is 0.4 mm, *b* is 0, and *c* is 1 mm, that is, *y* = 0.4*x*² *+* 0*x* + 1.

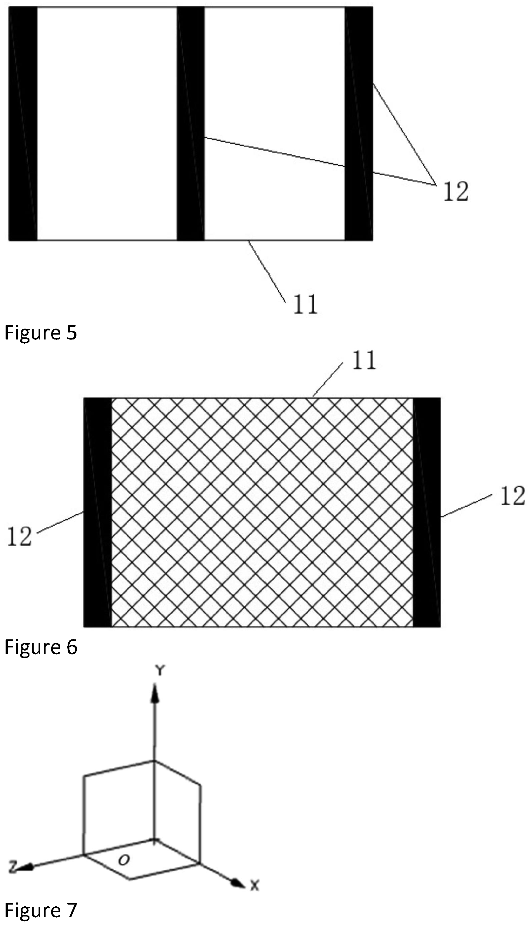

In addition, a rotation angle *α* between a projection of the boundary line or the local boundary line in a plane *-yOz* of a three-dimensional Cartesian coordinate system *O-xyz* and a -z-axis is 0°-90°. As shown in FIG. 7, a *z*-axis of the three-dimensional Cartesian coordinate system *O-xyz* is a straight line perpendicular to the Cartesian coordinate system *xoy* and passing through an origin O thereof, and the three-dimensional Cartesian coordinate system is a right-hand coordinate system.

For example, the boundary line may be a symmetrical center line or an outer contour line of the entire main headstrap 101. When the boundary line is the symmetrical center line of the entire main headstrap 101, the entire main headstrap 101 is obtained by extending outward from both sides of the boundary line; and when the boundary line is the outer contour line of the entire main headstrap 101, the entire main headstrap 101 is obtained by extending outward from one side of the boundary line.

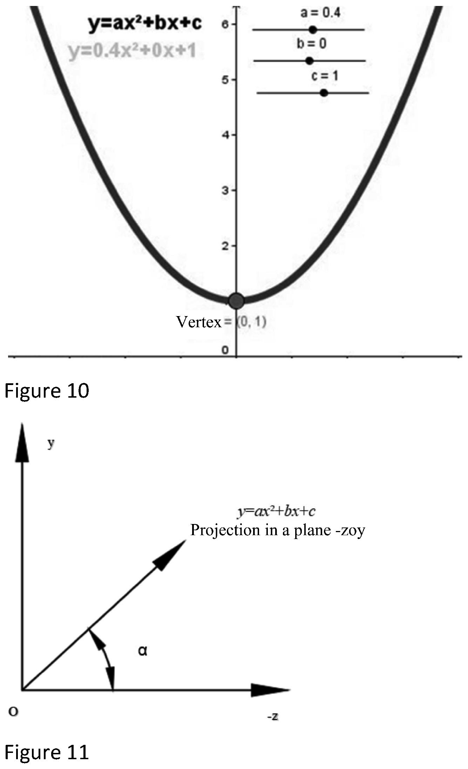

With reference to FIG. 10 and FIG. 1, the lowest point 103 of the main headstrap 101 may be, for example, the lowest point of the boundary line, i.e., the position of a point (0, 1) shown in FIG. 10. Therefore, it can be understood that the axis of symmetry of the main headstrap 101 passes through the lowest point 103.

Similarly, the local boundary line is a symmetrical center line or an outer contour line of at least one local strap among a plurality of local straps. When the local boundary line is the symmetrical center line of the local strap, the local strap is obtained by extending outward from both sides of the local boundary line; and when the local boundary line is the outer contour line of the local strap, the local strap is obtained by extending outward from one side of the local boundary line.

In addition, the local straps may be connected to each other by seams or integral molding, and the hardness of connection positions between the plurality of local straps is larger than the hardness of non-connection position. For example, the local straps are connected to each other by seams. Such a headgear assembly is generally formed of fabric, so it is necessary to increase the seam density at seam positions between the plurality of local straps, so that the hardness at the seam positions between the plurality of local straps is larger than that at non-seam positions, so as to avoid winding and bending at the seam positions, thereby preventing the service life thereof from being affected.

Alternatively, the local straps are connected to each other by means of integral molding. Such a headgear assembly is generally formed of a flexible material such as silica gel, so it is necessary to provide a connecting layer at the connection positions between the plurality of local straps, so that the hardness at the seam positions between the plurality of local straps is larger than that at the non-seam positions. The hardness of the connecting layer may be larger than that of the silica gel forming the headgear assembly, so as to avoid winding and bending at the seam positions, thereby preventing the service life thereof from being affected.

A plurality of connecting straps 102 may be provided. As shown in FIG. 1, the number of the connecting straps 102 may be two. Alternatively, as shown in FIG. 2, the number of the connecting straps 102 may also be four. It can be understood that the connecting straps 102 may be configured at free ends of the main headstrap 101, and are fixedly connected by means of hook-and-loop fasteners or magnetic snaps.

Specifically, as shown in FIG. 1, when the headgear assembly 1 is applied to a ventilation mask system such as a nasal mask, two connecting straps 102 may be configured on an entity of the entire headgear, and the two connecting straps 102 are respectively connected to a mask body 2 of the nasal mask (for example, beam arms on both sides of a frame). When the headgear assembly is applied to a ventilation mask system such as a full-face mask or an oronasal mask, four connecting straps 102 may be configured on the entity of the entire headgear (as shown in FIG. 2). Two of the four connecting straps 102 are connected to one side of the mask body 2, and the other two of the four connecting straps 102 are connected to the other side of the mask body 2.

Further, as shown in FIG. 1, the headgear assembly further includes a top headstrap 104, and both ends of the top headstrap 104 may be respectively connected to the main headstrap 101. The top headstrap 104 extends from the top of the user's head to both sides of the user's face when worn, thereby securing the headgear assembly at the top of the user's head. Therefore, the top headstrap 104 has a highest point, which may correspond to a middle position of the top of the user's head, for example.

The top headstrap 104 may be configured in a manner similar to that of the main headstrap 101. For example, the top headstrap 104 is integrally of a symmetrical structure, or at least part of the local straps on the main headstrap 101 are each of a symmetrical structure, and the symmetrical structure may have a boundary line or a local boundary line configured as one of the parabola, hyperbola, circle, and ellipse described above.

As shown in FIG. 1, the boundary line 13 is an outer contour line of the entire main headstrap 101 (a lower edge line shown in FIG. 1).

The shape of the headgear assembly formed by connecting the local straps has consistent convergence and divergence directions, avoiding the formation of curvature shapes that offset each other.

As shown in FIG. 1 and FIGS. 2-6, the strap body 11 (including the main headstrap 101, the connecting straps 102, and the top headstrap 104 described above) is formed of a fabric material. The fabric material is generally nylon or spandex, and a sandwich of other materials such as nitrile rubber, hollow cloth, or sponge may be added in the middle, which is formed as a whole by flame lamination or adhesive lamination.

The strap body 11 (including the main headstrap 101, the connecting straps 102, and the top headstrap 104 described above) is formed of a flexible material. The flexible material is, for example, a plastic material or a silica gel material. The plastic material generally refers to various types of plastic materials, which are combined with a fabric cover and made into a whole through assembly or integral molding; and the silica gel material generally refers to various types of silica gel materials, which are combined with a fabric cover and formed into a whole through assembly or adhesion.

The strap body 11 (including the main headstrap 101, the connecting straps 102, and the top headstrap 104 described above) is formed of a foam member including a sponge material. The foam member is a component made of a sponge material. The sponge is a porous material usually made of polyurethane or other synthetic materials, and has good breathability and softness. Therefore, the strap body 11 has good breathability and softness. However, the strength of the foam member does not meet the use requirements of the headgear assembly 1, and the strength includes tensile strength, breaking strength, etc.

The foam member has a reinforcing portion 12 formed by hot pressing. As shown in FIGS. 2-6, the strap body 11 is a foam member, a reinforcing portion 12 is provided thereon, and the reinforcing portion 12 is formed by hot pressing. Hot pressing is a process that uses high temperature and high pressure to change the properties or shape of materials. The reinforcing portion 12 is formed on the foam member by hot pressing, so that the strap body 11 has good softness, breathability, and sufficient strength at the same time.

The strap body 11 being a foam member enables the strap body 11 to have good breathability and softness, and the reinforcing portion 12 provided on the strap body 11 can increase the strength of the strap body 11, so that the headgear assembly 1 has sufficient strength while maintaining good softness and breathability. That is, the contact between the headgear assembly 1 and the user can improve the user's comfort, while the strength requirement is met to support the mask body on the user's face.

The softness and breathability of the headgear assembly 1 are main performances for improving comfort and usability, and the strength is to maintain the durability of the headgear assembly 1.

Optionally or additionally, for example, the reinforcing portion 12 may be disposed on at least one end of the strap body 11 in a width direction of the strap body 11, and the reinforcing portion 12 extends in a length direction of the strap body 11.

Referring to FIG. 4, the width direction of the strap body 11 is an arrow direction A, and the length direction of the strap body 11 is an arrow direction B. A reinforcing portion 12 is provided at one end of the strap body 11 in the width direction; alternatively, reinforcing portions 12 are provided at both ends of the strap body 11 in the width direction, respectively.

When the reinforcing portion 12 is disposed on at least one end of the strap body 11 in the width direction, the reinforcing portion 12 increases the strength of the strap body 11 without affecting the breathability of portions of the strap body 11 except for the reinforcing portion 12, thereby enabling the headgear assembly 1 to have better breathability. Moreover, the strap body 11 is stretched along the length direction during stretching, and the reinforcing portion 12 extending along the length direction of the strap body 11 can effectively improve the tensile strength of the strap body 11.

Optionally or additionally, at least one reinforcing portion 12 is disposed in a middle portion of the strap body 11 along the width direction, and the reinforcing portion 12 extends along the length direction of the strap body 11.

Referring to FIG. 5, one reinforcing portion 12 is disposed in the middle portion of the strap body 11 along the width direction; alternatively, at least two reinforcing portions 12 may be disposed in the middle portion of the strap body 11 along the width direction, and the at least two reinforcing portions 12 are spaced apart along the width direction.

Providing at least one reinforcing portion 12 in the middle portion of the strap body 11 along the width direction can effectively increase the strength of the strap body 11, so that the strap body 11 meets the strength requirement. The reinforcing portion 12 extending along the length direction of the strap body 11 can also effectively improve the tensile strength of the strap body 11.

Optionally or additionally, a reinforcing portion 12 is provided on a front surface of the strap body 11. The front surface of the strap body 11 refers to a surface of the strap body 11 that faces away from the user when in use, and a back surface of the strap body 11 refers to a surface of the headgear assembly 1 that is in contact with the user.

Since the part of the strap body 11 without the reinforcing portion 12 has better softness and breathability than the reinforcing portion 12, the reinforcing portion 12 is disposed on the front surface of the strap body 11, and no reinforcing portion 12 is disposed on the back surface of the strap body 11, which can effectively improve the user's comfort when the headgear assembly 1 is in contact with the user.

Optionally or additionally, in the thickness direction of the strap body 11, the strap body 11 includes at least one layer of foam member, and a density of the at least one layer of foam member is greater than or equal to 45 Kg/m³. When the strap body 11 includes at least one layer of foam member, the number of layers of the foam member and the corresponding density may be set according to the requirements of softness, breathability, and strength to meet the use requirements of different headgear assemblies 1. For example, the strap body 11 includes a first foam member, and the density of the first foam member is greater than or equal to 45 Kg/m³. When the density of the first foam member is within the above range, the reinforcing portion 12 formed on the first foam member by hot pressing can meet the strength requirement of the headgear assembly 1.

Optionally or additionally, in the thickness direction of the strap body 11, the strap body 11 includes at least two layers of foam members, and the at least two layers of foam members are arranged in a stacked manner in the thickness direction of the strap body 11.

The thickness direction of the strap body 11 is perpendicular to the arrow direction A and the arrow direction B, respectively. When the strap body 11 includes at least two layers of foam members, the foam members may have various densities. For example, foam members with different densities have different strengths, and reinforcing portions 12 formed by hot pressing also have different strengths.

For example, the strap body 11 includes a first foam member and a second foam member, the first foam member is located on the front surface of the strap body 11, the second foam member is located on the back surface of the strap body 11, and the density of the first foam member is larger than that of the second foam member. That is, the strap body 11 is formed by stacking the first foam member and the second foam member in the thickness direction of the strap body 11, which can meet the requirements of the headgear assembly 1 for softness, breathability, and strength. The density of the first foam member is larger than that of the second foam member, the first foam member has better strength, and the first foam member is located on the front surface of the strap body 11, which can increase the strength of the strap body 11. The second foam member is located on the back surface of the strap body 11 and is in contact with the user. The density of the second foam member is lower than that of the first foam member, which has better breathability and softness, effectively improving the user's comfort.

Optionally or additionally, in the thickness direction of the strap body 11, the strap body 11 includes at least three layers of foam members. For example, the strap body 11 includes a first foam member, a second foam member, and a third foam member. The first foam member is located on the front surface of the strap body 11, the second foam member is located on the back surface of the strap body 11, and the third foam member is located between the first foam member and the second foam member. The foam member has at least three layers of foam members, which makes the design of the foam member more flexible to meet different needs for breathability, softness, and strength.

The density of the third foam member is larger than that of the second foam member, and the density of the first foam member is larger than that of the second foam member.

The foam member is made of a material with good softness and breathability, and the foam member, as an independent headgear assembly 1, can greatly improve the comfort of the headgear assembly 1. The provision of the reinforcing portion 12 on the foam member can make the foam member meet the use requirements for strength, thereby improving the usability of the headgear assembly. According to the structure, the foam members may generally be divided into high-density sponge, medium-density sponge, and low-density sponge. The high-density sponge has a density greater than or equal to 45 Kg/m³, the medium-density sponge has a density of 18 Kg/m³-45 Kg/m³, and the low-density sponge has a density less than 18 Kg/m³.

During the manufacturing process of the strap body 11, the first foam member, the second foam member, and the third foam member with corresponding densities may be selected according to the use requirements. By selecting different foam member densities, different foam member thicknesses, and different hot pressing positions, independent foam members with the strength meeting the use requirements may be combined as the strap body 11 of the headgear assembly 1.

Further, in order to improve the strength of the strap body 11, the hot pressing area can be increased. Of course, the increase in the hot pressing area will reduce the breathability of the strap body 11, because the area after hot pressing may change an original form of the sponge and be closer to a dense body.

Moreover, using two or more types of foam members, the strap body 11 with a plurality of foam members is formed at the ends in the width direction by hot pressing. The foam member on the back surface of the strap body 11 can use a low-density sponge with a relatively low density to improve softness and breathability, and the foam member on the front surface of the strap body 11 can appropriately increase the sponge density to enhance the strength of the headgear assembly.

By combining the plurality of foam members, different sponge densities, different hot pressing areas, and improving the local strength and overall strength of the foam members, a more suitable headgear assembly can be adjusted. The foam member is suitable as an independent material for the headgear assembly 1 to meet the requirements of comfort and usability.

The thickness of the strap body 11 ranges from 3 mm to 6 mm. The thickness of the strap body 11 within the above range can not only meet the requirements of the headgear assembly 1 for breathability, softness, and tensile strength, but also avoid the bulkiness caused by an excessive thickness.

The tensile strength of the strap body 11 ranges from 450 Kpa to 900 Kpa. The tensile strength of the strap body 11 within the above range can meet the use requirements after the headgear assembly 1 is connected to the mask.

Referring to FIG. 6, the back surface of the strap body 11 is provided with a grid texture, which can increase the friction between the strap body 11 and the user to avoid random shaking of the headgear assembly 1 during use.

Referring to FIG. 2, the headgear assembly 1 includes at least one strap body 11. When the headgear assembly 1 includes at least two strap bodies 11, the at least two strap bodies 11 are spliced. That is, the headgear assembly 1 includes one headstrap unit, or the headgear assembly 1 includes at least two strap bodies 11; and when the headgear assembly 1 includes at least two strap bodies 11, the at least two strap bodies 11 are connected to meet different styling requirements of the headgear assembly 1.

The headgear assembly of the present disclosure may be obtained by implementing the following manufacturing method for the headgear assembly. Alternatively, the headgear assembly of the present disclosure may also be implemented independently.

The present disclosure also provides a ventilation mask system, which includes a mask body and the headgear assembly 1 described above, and the mask body 2 is connected to the headgear assembly 1.

The mask body 2 includes a mask shell, a mask sealing member, and the like. The mask body may be a nasal mask covering only the nose, an oral mask covering only the mouth, an oronasal mask (also referred to as a full-face mask) covering both the mouth and the nose, a nasal pad mask inserted into nostrils, and a ventilation mask in the form of a ventilation headstrap tube 20 as shown in FIG. 3, where the headgear assembly 1 (strap body 11) is wrapped around at least a portion of an outer surface of the ventilation headstrap tube 20.

The mask body is worn on the user's face, and the mask body is positioned at a relatively comfortable position on the face by means of the headgear assembly, thereby ensuring the comfort of wearing the mask body and further better realizing the regulation, improvement, and therapeutic functions of the mask body. The headgear assembly 1 is a main component connecting the mask body to the user's head. Due to large individual differences in head shapes, the headgear assembly 1 needs to have good comfort, conformability, and sufficient adaptability.

The strap body 11 of the headgear assembly 1 being a foam member enables the strap body 11 to have good breathability and softness, and the reinforcing portion 12 provided on the strap body 11 can increase the strength of the strap body 11, so that the headgear assembly 1 has sufficient strength while maintaining good softness and breathability, which can improve the user's experience and better meet the usability requirements.

The mask system is suitable for application in a ventilation apparatus, which includes a ventilator and the above-mentioned mask system, and the ventilator is connected to the mask system.

It should be noted that the ventilation apparatus, the mask system, and the headgear assembly 1 can refer to each other, and have the same or similar beneficial effects as any of the headgear assemblies 1 described above. To avoid repetition, details are not repeated here.

As shown in FIG. 1, the headgear assembly 1 may be connected to the mask body 2 via the connecting straps 102, for example. The mask body 2 includes a frame, cushions respectively located on both sides of the frame (not shown), and a ventilation gas pipeline 3, where the frame is fixedly connected or detachably connected to the cushions, and the ventilation gas pipeline 3 is connected to the frame or the cushions. The ventilation gas pipeline 3 is in fluid communication with the frame and the cushions, so that a ventilation gas can be delivered to the user for the user's breathing. The headgear assembly 1 can secure the mask body on the user's face, thereby ensuring the sealing between the mask body and the user's face.

The present disclosure also provides a manufacturing method for a headgear assembly, including the following steps:
100: Construct a mathematical model of a headgear assembly.

Specifically, step 100 includes the following sub-steps:
110: Establish a Cartesian coordinate system *xOy.*

Two number axes that are perpendicular to each other and have a common origin on the same plane form a Cartesian coordinate system, which is referred to as rectangular coordinates. Generally, the two number axes are placed in horizontal and vertical positions, respectively, and the directions to the right and upward are taken as positive directions of the two number axes, respectively. The horizontal number axis is the *x*-axis or a horizontal axis, the vertical number axis is the *y*-axis or a vertical axis, the *x*-axis and *y*-axis are collectively referred to as coordinate axes, their common origin *O* is called as the origin of the Cartesian coordinate system, and the Cartesian coordinate system with point *O* as the origin is denoted as the Cartesian coordinate system *xOy.*

As shown in FIGS. 7-9, the origin *O* of the Cartesian coordinate system *xOy* is a midpoint M₂ (as shown in FIG. 9) of a symmetrical part of the user's neck (the axis of symmetry is shown as a dash-dot line in FIG. 9), the *y*-axis of the Cartesian coordinate system *xOy* is a connecting line between the highest point M₁ (as shown in FIG. 8) on the occipital region of the user and the midpoint M₂, and the x-axis of the Cartesian coordinate system *xOy* is a straight line passing through the origin *O* and perpendicular to the *y*-axis.

120: Establish a three-dimensional Cartesian coordinate system *O-xyz.*

Arbitrarily select a point *O* in space, and draw three mutually perpendicular number axes *Ox, Oy,* and *Oz* via the point *O*, all of which have the same unit length with *O* as the origin. These three axes are respectively the x-axis (horizontal axis), *y*-axis (vertical axis), and *z*-axis (longitudinal axis), which are collectively referred to as coordinate axes. Their positive directions conform to the right-hand rule, that is, when holding the z-axis with the right hand, and four fingers of the right hand turn from the positive direction of the x-axis to the positive direction of the *y*-axis at an angle of $\frac{π}{2}$, the direction pointed by the thumb is the positive direction of the z-axis. This constitutes a three-dimensional Cartesian coordinate system, which is called as the three-dimensional Cartesian coordinate system *O-xyz.* The fixed point *O* is called as the origin of the coordinate system.

As shown in FIG. 7, the z-axis of the three-dimensional Cartesian coordinate system *O-xyz* is a straight line perpendicular to the Cartesian coordinate system *xOy* and passing through the origin *O*, and the three-dimensional Cartesian coordinate system is a right-hand coordinate system.

130: Construct a boundary line of an entire headgear or a local boundary line of at least one local strap among a plurality of unitized local straps in the Cartesian coordinate system *xOy.* The boundary line or the local boundary line is one of a parabola, a hyperbola, a circle, and an ellipse in the Cartesian coordinate system *xOy.*

Specifically, the boundary line or the local boundary line may be a parabola, for example, which satisfies the following relational expression:
$y = {ax}^{2} + bx + c ,$
where *y* is a value on the *y*-axis in the Cartesian coordinate system *xOy* for the boundary line, and *x* is a value on the *x*-axis in the Cartesian coordinate system *xOy* for the boundary line. *a, b* and *c* are all constants, 0 *< a* < 1.2 mm, *b =* 0, and 0 ≤ *c* ≤ 1.2 mm. As shown in FIG. 10, the functional relational expression of the boundary line or the local boundary line is shown when *a* is 0.4 mm, *b* is 0, and *c* is 1 mm, that is, *y* = 0.4*x*² *+* 0*x* + 1.

Further, as shown in FIG. 5, the rotation angle *α* between the projection of the boundary line or the local boundary line in the plane *-yOz* of the three-dimensional Cartesian coordinate system *O-xyz* and the -*z* axis is 0°-90°.

Therefore, by establishing the Cartesian coordinate system *xOy* and the three-dimensional Cartesian coordinate system *O-xyz* with reference to the structure of the user's head, the mathematical model of the headgear assembly can be constructed, thereby providing quantifiable and visualized reliable data for the production of the headgear assembly, reducing dimensional differences of the headgear assembly, and realizing the unification of main dimensions of the headgear assembly. In addition, the mathematical model of the headgear assembly can be used as the main shape of the headgear assembly, and auxiliary structures may be added on the basis of the main shape to make the headgear more individualized in design.

In addition, the boundary line or the local boundary line may also be a hyperbola, which satisfies the following relational expression:
$\frac{{x}^{2}}{{a}^{2}} - \frac{{y}^{2}}{{b}^{2}} = 1 ;$
or satisfies the following relational expression:
$\frac{{y}^{2}}{{a}^{2}} - \frac{{x}^{2}}{{b}^{2}} = 1 ;$
where *a* and *b* in formulas (1) and (2) are both constants greater than 0, the foci in formula (1) are (-c, 0) and (c, 0), and the foci in formula (2) are (0, -c) and (0, c).

In addition, the boundary line or the local boundary line may also be a circle, which satisfies the following relational expression:
${\left(x-a\right)}^{2} + {\left(y-b\right)}^{2} = {r}^{2} ;$
where *a* and *b* are both constants, and *r* is the radius of the circle.

In addition, the boundary line or the local boundary line may also be an ellipse, which satisfies the following relational expression:
$\frac{{x}^{2}}{{a}^{2}} + \frac{{y}^{2}}{{b}^{2}} = 1 ;$
where *a* and *b* are both constants greater than 0.

200: Materialize the mathematical model to obtain an entity of the entire headgear or an entity of at least one local strap among the plurality of unitized local straps.

Specifically, in step 200, materializing the mathematical model to obtain the entity of the entire headgear includes the following steps:
210: With the boundary line as the symmetrical center line, extend to both sides of the boundary line respectively to obtain the entity of the entire headgear; or with the boundary line as the outer contour line, extend to one side of the boundary line to obtain the entity of the entire headgear.

In addition, materializing the mathematical model in step 200 to obtain the entity of at least one local strap among the plurality of unitized local straps includes the following steps:
220: With the local boundary line as the symmetrical center line, extend to both sides of the local boundary line respectively to obtain the entity of at least one local strap among the plurality of local straps; or with the local boundary line as the outer contour line, extend to one side of the local boundary line to obtain the entity of at least one local strap among the plurality of local straps.

Further, during the process of materializing the mathematical model to obtain the entity of at least one local strap among the plurality of unitized local straps, the shape formed by connecting the local straps shall not form a curvature shape that offsets each other, but the entire shape shall have consistent convergence and divergence directions.

230: Connect the materialized local straps to each other.

Since each unitized local strap can be a portion of the entire headgear, the entire headgear can be formed by connecting the unitized local straps to each other. Therefore, it is necessary to connect the materialized local straps to each other.

For example, the local straps are connected to each other by seams. Such a headgear assembly is generally formed of fabric, so it is necessary to increase the seam density at seam positions between the plurality of local straps, so that the hardness at the seam positions between the plurality of local straps is larger than that at non-seam positions, so as to avoid winding and bending at the seam positions, thereby preventing the service life thereof from being affected.

Alternatively, the local straps are connected to each other by means of integral molding. Such a headgear assembly is generally formed of a flexible material such as silica gel, so it is necessary to provide a connecting layer at the connection positions between the plurality of local straps, so that the hardness at the seam positions between the plurality of local straps is larger than that at the non-seam positions. The hardness of the connecting layer may be larger than that of the silica gel forming the headgear assembly, so as to avoid winding and bending at the seam positions, thereby preventing the service life thereof from being affected.

300: Construct connecting straps for connecting to the mask body of the ventilation mask system on the entity of the entire headgear or on the entity of at least one local strap among the plurality of unitized local straps, respectively. For example, two or four connecting straps may be formed on the entity of the entire headgear, thereby forming a two-point headgear assembly or a four-point headgear assembly.

Specifically, when the headgear assembly is applied to a ventilation mask system such as a nasal mask, two connecting straps may be configured on the entity of the entire headgear, and the two connecting straps are respectively connected to the beam arms on both sides of the frame of the nasal mask. When the headgear assembly is applied to a ventilation mask system such as a full-face mask or an oronasal mask, four connecting straps may be configured on the entity of the entire headgear. Two of the four connecting straps are respectively connected to one side of the mask body, and the other two of the four connecting straps are respectively connected to the other side of the mask body.

Therefore, by controlling a curvature direction of the mathematical models of the plurality of unitized local straps formed in step 100 to maintain consistent convergence and divergence directions, and processing the connection positions of the materialized local straps, a basis can be provided for promoting the automated production of the headgear assembly.

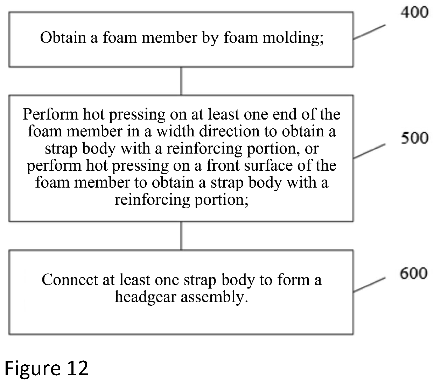

Optionally or additionally, the manufacturing method further includes a step of manufacturing the headgear assembly 1 based on the mathematical model of the headstrap described above, which includes the following operation steps as shown in FIG. 12.

400: Provide a foam member.

In this step, one layer or at least two layers of foam members may be provided. For example, the foam member is a first foam member, or the foam members include a first foam member and a second foam member arranged in a stacked manner along the thickness direction. Moreover, the foam member on the back surface of the strap body 11 may adopt a low-density sponge with a relatively low density to improve softness and breathability, and the foam member on the front surface of the strap body 11 may appropriately increase the sponge density to enhance the strength of the headgear assembly.

The foam member may be formed by foam molding, that is, an external mold for the headgear assembly 1 is made, and the external shape of the headgear assembly 1 is processed by foam molding.

500: Perform hot pressing on at least part of the area of the foam member to form a reinforcing portion 12, so as to manufacture the strap body 11.

In this step, the strap body 11 can be manufactured by performing hot pressing on at least part of the area of the foam member to form the reinforcing portion 12.

Optionally or additionally, the step of performing hot pressing on at least part of the area of the foam member to form the reinforcing portion 12 includes performing hot pressing on at least one end of the foam member along the width direction to form the reinforcing portion 12. The width direction of the foam member is the same as the width direction of the strap body 11.

At this time, in addition to increasing the strength of the strap body 11, the reinforcing portion can also assist in the connection of at least two layers of foam members.

Optionally or additionally, the step of performing hot pressing on at least part of the area of the foam member to form the reinforcing portion 12 includes performing hot pressing on the front surface of the foam member to form the reinforcing portion 12.

The reinforcing portion 12 is disposed on the front surface of the strap body 11, and no reinforcing portion 12 is disposed on the back surface of the strap body 11, which can reduce the impact of the reinforcing portion 12 on the softness and breathability of the back surface of the strap body 11, and the headgear assembly 1 has better comfort when in contact with the user.

The reinforcing portion 12 can improve the strength of the foam member. For example, high-density sponge has higher tensile strength than low-density sponge, and the tensile strength of the high-density sponge is greatly improved after hot pressing, for example, increased by 3-6 times. For example, the tensile strength range of the high-density sponge is less than or equal to 150 KPa, and after local hot pressing, the tensile strength range can reach 450 KPa-900 KPa, which is sufficient for the mask body to be worn and used within an appropriate pressure range.

600: Connect at least one strap body 11 to form the headgear assembly.

In this step, when the headgear assembly 1 includes one headstrap unit, the headstrap unit is connected to the mask body. When the headgear assembly 1 includes at least two strap bodies 11, the at least two strap bodies 11 are spliced and then connected to the mask body. The above structure can meet different styling requirements of the headgear assembly 1.

For the headgear assembly manufactured by the manufacturing method of the present disclosure, the strap body 11 being a foam member enables the strap body 11 to have good breathability and softness, and the reinforcing portion 12 provided on the strap body 11 can increase the strength of the strap body 11, so that the headgear assembly 1 has sufficient strength while maintaining good softness and breathability. That is, the contact between the headgear assembly 1 and the user can improve the user's comfort, while the strength requirement is met to support the mask body on the user's face.

It should be noted that in this specification, relational terms such as "first" and "second" are only used to distinguish one entity or operation from another entity or operation, and do not necessarily require or imply that there is any such actual relationship or order between these entities or operations. Moreover, the terms "comprise", "include", or their any other variants are intended to cover a non-exclusive inclusion, so that a process, a method, an article, or a device that includes a list of elements not only includes those elements but also includes other elements which are not expressly listed, or further includes elements inherent to such process, method, article, or device. If no more limitations are made, an element limited by "including a/an ..." does not exclude other identical elements existing in the process, the method, the article, or the device which includes the element.

The embodiments in the specification are all described in a relevant manner, mutual reference may be made to the same or similar parts of the embodiments, and each embodiment focuses on description of differences from other embodiments. As the embodiments of an apparatus, an electronic device, a computer-readable storage medium and a computer program product containing instructions are substantially similar to method embodiments, their descriptions are relatively concise, and for relevant details, reference may be made to the corresponding descriptions in the method embodiments.

Although the present disclosure has been described with reference to preferred embodiments, various improvements can be made thereto and equivalents can be used for the replacement of components therein without departing from the scope of the present disclosure. In particular, as long as there is no structural conflict, the technical features mentioned in each of the embodiments can be combined in any manner. The present disclosure is not limited to the specific embodiments disclosed herein but includes all the technical solutions that fall within the scope of the claims.

## Claims

1. A headgear assembly for securing a mask body (2) of a ventilation mask system to the user's head, the headgear assembly (1) comprising a strap body (11), the strap body (11) comprising a main headstrap (101) and connecting straps (102) connected to the main headstrap (101), the main headstrap (101) being located at an occipital region of the user when worn, and the connecting straps (102) being configured to connect to the mask body (2) of the ventilation mask system,
**characterized in that** the main headstrap (101) has a lowest point and is recessed upward and toward the connecting straps (102) from the lowest point, wherein the main headstrap (101) is integrally of a symmetrical structure, or at least part of local straps on the main headstrap (101) are each of a symmetrical structure, and the symmetrical structure has a boundary line or a local boundary line configured as one of a parabola, a hyperbola, a circle, and an ellipse.

2. The headgear assembly according to claim 1, **characterized in that** the boundary line is a symmetrical center line or an outer contour line of the entire main headstrap; or
the local boundary line is a symmetrical center line or an outer contour line of at least part of the local straps on the main headstrap.

3. The headgear assembly according to claim 1 or 2, **characterized in that** the strap body (11) is formed of a fabric material, a flexible material, or a foam member comprising a sponge material, wherein the foam member has a reinforcing portion (12) formed by hot pressing.

4. The headgear assembly according to claim 3, **characterized in that** the reinforcing portion (12) is disposed at any one of the following positions:
at least one end of the strap body (11) in a width direction, the reinforcing portion extending in a length direction of the strap body (11);
a middle portion of the strap body (11) in the width direction, the reinforcing portion extending in the length direction of the strap body (11); and
a front surface of the strap body (11).

5. The headgear assembly according to claim 3 or 4, **characterized in that** in a thickness direction of the strap body (11), the strap body (11) comprises at least one layer of foam member, and a density of the at least one layer of foam member is greater than or equal to 45 Kg/m³; or
in the thickness direction of the strap body (11), the strap body (11) comprises at least two layers of foam members, the at least two layers of foam members are arranged in a stacked manner in the thickness direction, and the density of the foam member located on a front surface of the strap body (11) among the at least two layers of foam members is larger than the density of the foam member located on a back surface of the strap body (11).

6. The headgear assembly according to any one of claims 3-5, **characterized in that** the strap body (11) comprises a first foam member located on a front surface of the strap body (11), a second foam member located on a back surface of the strap body (11), and a third foam member located between the first foam member and the second foam member, wherein the density of the third foam member is larger than that of the second foam member, and the density of the first foam member is larger than that of the second foam member.

7. The headgear assembly according to any one of claims 1-6, **characterized in that** a thickness of the strap body (11) ranges from 3 mm to 6 mm; and/or
the tensile strength of the strap body (11) ranges from 450 Kpa to 900 Kpa.

8. The headgear assembly according to any one of claims 1-7, **characterized in that** the local straps on the main headstrap (101) are connected to each other by seams or integral molding, and the hardness of connection positions between the local straps is larger than the hardness of non-connection positions.

9. The headgear assembly according to any one of claims 1-8, **characterized in that** two or four connecting straps (102) are provided, and the main headstrap (101) and the respective connecting straps (102) are connected to each other by seams or integral molding.

10. The headgear assembly according to any one of claims 1-9, **characterized in that** the strap body (11) is configured to be wrapped around a ventilation headstrap tube (20) of the mask body (2).

11. The headgear assembly according to any one of claims 1-10, **characterized in that** the boundary line or the local boundary line satisfies the following relational expression:$y = {ax}^{2} + bx + c ,$
wherein *y* is a value on a *y*-axis in a Cartesian coordinate system *xOy* for the boundary line, and *x* is a value on an *x*-axis in the Cartesian coordinate system *xOy* for the boundary line; and
*a, b* and *c* are all constants, 0 *< a* < 1.2 mm, *b =* 0, and 0 ≤ *c* ≤ 1.2 mm.

12. The headgear assembly according to claim 11, **characterized in that** a rotation angle *α* between a projection of the boundary line or the local boundary line in a *-yOz* plane of a three-dimensional Cartesian coordinate system *O - xyz* and a -*z* axis is 0°-90°, wherein a *z*-axis of the three-dimensional Cartesian coordinate system *O - xyz* is a straight line perpendicular to the Cartesian coordinate system *xoy* and passing through an origin *O* of the Cartesian coordinate system *xoy,* and the three-dimensional Cartesian coordinate system is a right-hand coordinate system.

13. A ventilation mask system, **characterized by** comprising a headgear assembly according to any one of claims 1-12, and further comprising a mask body (2), the strap body (11) being connected to the mask body (2).

14. A manufacturing method for a headgear assembly, **characterized by** comprising a step of constructing a mathematical model of a main headstrap of a headgear assembly, the step of constructing the mathematical model of the main headstrap of the headgear assembly comprising the following steps:
establishing a Cartesian coordinate system *xOy,* wherein an origin *O* of the Cartesian coordinate system *xOy* is a midpoint of a symmetrical part of the user's neck, a *y*-axis of the Cartesian coordinate system *xOy* is a connecting line between a highest point on an occipital region of the user and the midpoint, and an *x*-axis of the Cartesian coordinate system *xOy* is a straight line passing through the origin *O* and perpendicular to the *y*-axis; and
constructing a boundary line of the entire main headstrap or a local boundary line of at least one local strap among a plurality of unitized local straps in the Cartesian coordinate system *xOy,*
wherein the boundary line or the local boundary line is one of a parabola, a hyperbola, a circle, and an ellipse in the Cartesian coordinate system *xOy.*

15. The manufacturing method for a headgear assembly according to claim 14, **characterized by** further comprising a step of manufacturing the headgear assembly (1) according to the mathematical model of the headstrap, the step comprising the following operation steps:
obtaining a foam member by foam molding;
performing hot pressing on at least one end of the foam member in a width direction to obtain a strap body (11) with a reinforcing portion (12), or performing hot pressing on a front surface of the foam member to obtain a strap body (11) with a reinforcing portion (12); and
connecting at least one strap body (11) to form the headgear assembly.
